# EUROPEAN PATENT APPLICATION

(11) **EP 3 977 927 A1**
(43) Date of publication of application: **06.04.2022**
(21) Application number: 19930851.1
(22) Date of filing: 29.05.2019
(51) Int. Cl.: A61B 5/117, A61B 5/11, G06F 21/32

(54) **INFORMATION PROCESSING DEVICE, PERSONAL IDENTIFICATION DEVICE, PERSONAL IDENTIFICATION SYSTEM, INFORMATION PROCESSING METHOD, AND RECORDING MEDIUM**

(71) Applicant: NEC Corporation, 108-8001 Tokyo (JP)
(72) Inventor: HUANG, Chenhui, Tokyo 108-8001 (JP); FUKUSHI, Kenichiro, Tokyo 108-8001 (JP); NAKAHARA, Kentaro, Tokyo 108-8001 (JP)
(74) Representative: Betten & Resch
(86) International application number: PCT/JP2019/021421
(87) International publication number: WO 2020/240750

(57) **Abstract**

Provided is an information processing device including an acquisition unit configured to acquire first load information measured by a first load measurement device provided on a sole of a user and second load information measured by a second load measurement device provided between a toe of the sole and the first load measurement device and a feature amount extracting unit configured to extract a feature amount used for identifying the user based on the first load information and the second load information.

## Description

### [Technical Field]

The present invention relates to an information processing device, a personal identification device, a personal identification system, an information processing method, and a storage medium.

### [Background Art]

Patent Literature 1 discloses a personal authentication system that performs personal authentication using a load distribution detected by a contact load distribution sensor mounted on a shoe.

### [Citation List]

### [Patent Literature]

PTL 1: Japanese Patent Application Laid-open No. 2008-250996

### [Summary of Invention]

### [Technical Problem]

Personal identification as disclosed in Patent Literature 1 requires acquiring and processing a lot of data for feature extraction. Therefore, reduction in the amount of data used for feature extraction is required.

The present invention intends to provide an information processing device, a personal identification device, a personal identification system, an information processing method, and a storage medium which can reduce the amount of data in extracting a feature amount used for personal identification.

### [Solution to Problem]

According to one example aspect of the invention, provided is an information processing device including an acquisition unit configured to acquire first load information measured by a first load measurement device provided on a sole of a user and second load information measured by a second load measurement device provided between a toe of the sole and the first load measurement device and a feature amount extracting unit configured to extract a feature amount used for identifying the user based on the first load information and the second load information.

According to another example aspect of the invention, provided is an information processing method including acquiring first load information measured by a first load measurement device provided on a sole of a user and second load information measured by a second load measurement device provided between a toe of the sole and the first load measurement device and extracting a feature amount used for identifying the user based on the first load information and the second load information.

According to another example aspect of the invention, provided is a storage medium storing a program that causes a computer to perform acquiring first load information measured by a first load measurement device provided on a sole of a user and second load information measured by a second load measurement device provided between a toe of the sole than the first load measurement device and extracting a feature amount used for identifying the user based on the first load information and the second load information.

### [Advantageous Effects of Invention]

According to the present invention, an information processing device, a personal identification device, a personal identification system, an information processing method, and a storage medium which can reduce the amount of data in extracting a feature amount used for personal identification can be provided.

### [Brief Description of Drawings]

[Fig. 1]

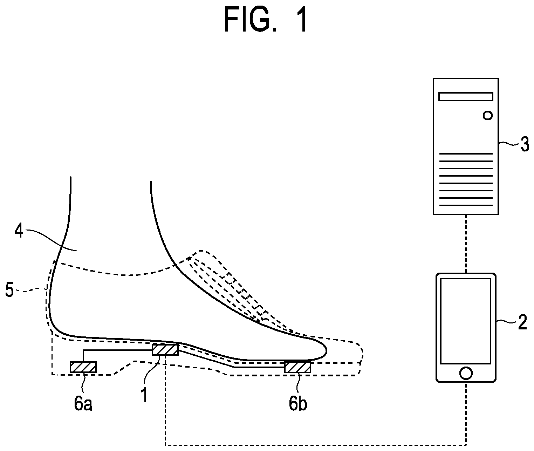

   Fig. 1 is a schematic diagram illustrating a general configuration of a personal identification system according to a first example embodiment.
[Fig. 2]

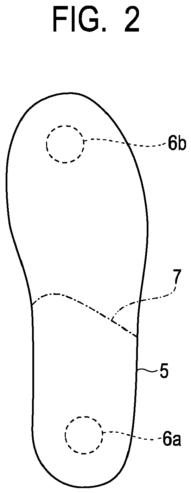

   Fig. 2 is a schematic diagram illustrating an arrangement of a load measurement device according to the first example embodiment.
[Fig. 3]

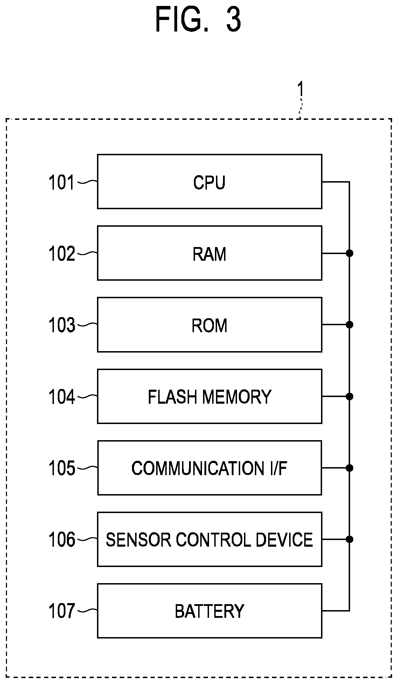

   Fig. 3 is a block diagram illustrating a hardware configuration of a gait measurement device according to the first example embodiment.
[Fig. 4]

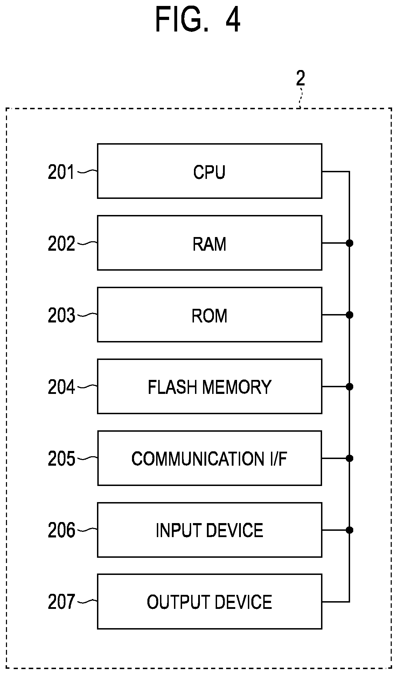

   Fig. 4 is a block diagram illustrating a hardware configuration of an information communication terminal according to the first example embodiment.
[Fig. 5]

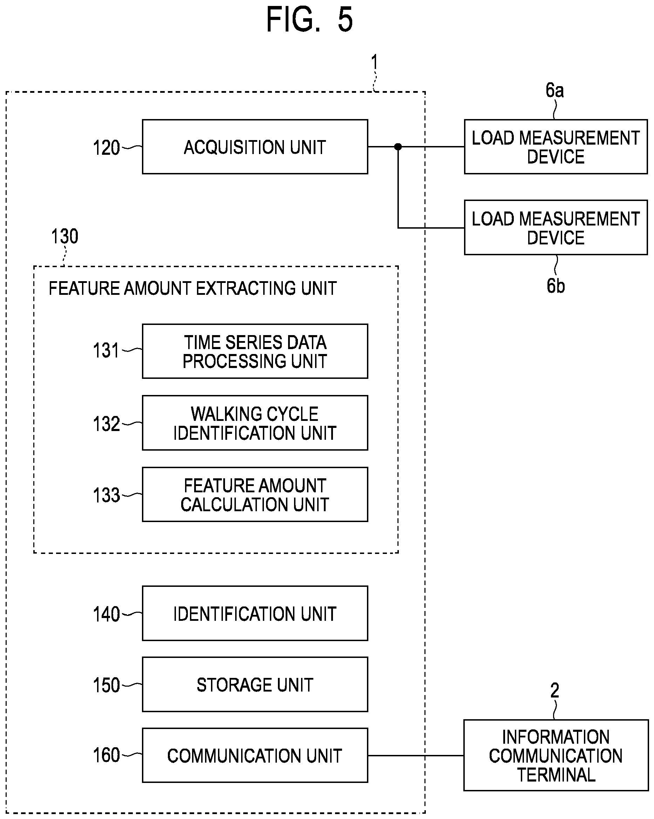

   Fig. 5 is a functional block diagram of a gait measurement device according to the first example embodiment.
[Fig. 6]

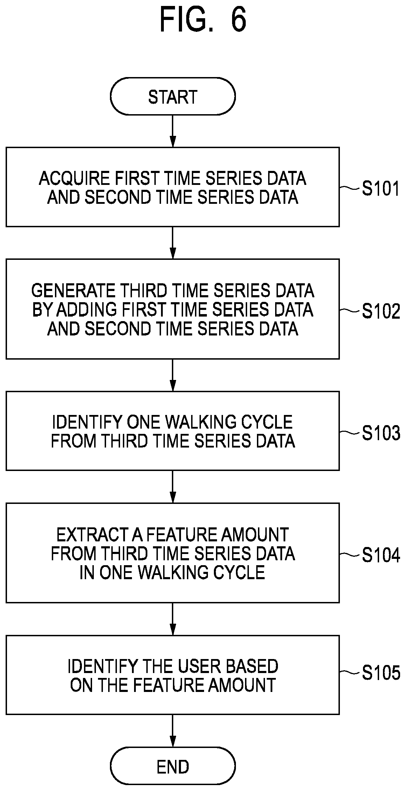

   Fig. 6 is a flowchart illustrating an example of personal identification processing performed by the gait measurement device according to the first example embodiment.
[Fig. 7]

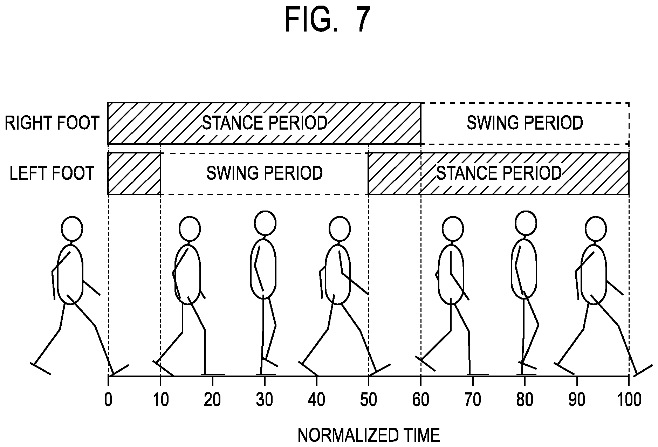

   Fig. 7 is a conceptual diagram illustrating a walking cycle.
[Fig. 8]

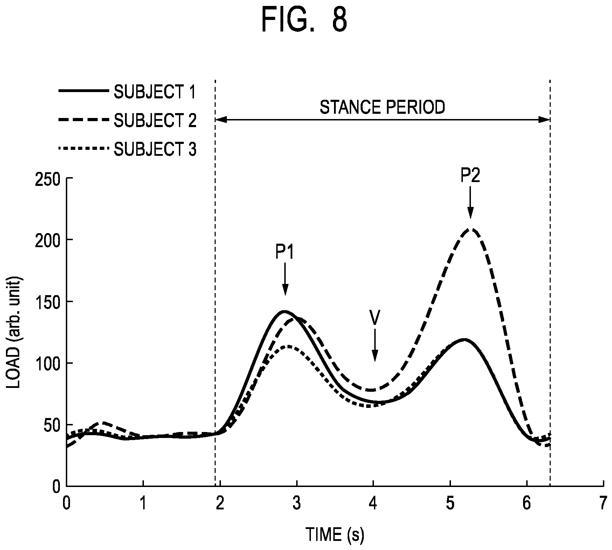

   Fig. 8 is a graph illustrating an example of time series data of the load.
[Fig. 9]

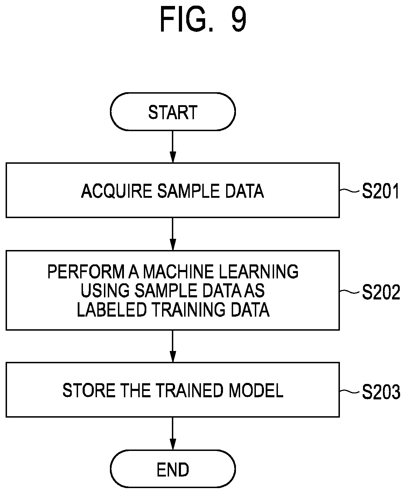

   Fig. 9 is a flowchart illustrating an example of training process performed by the server according to the first example embodiment.
[Fig. 10]

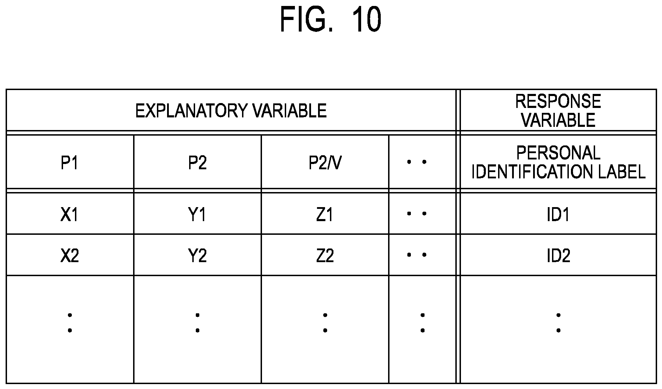

   Fig. 10 is a table schematically illustrating a correspondence relation between a feature amount vector and a personal identification label acquired by the training process.
[Fig. 11]

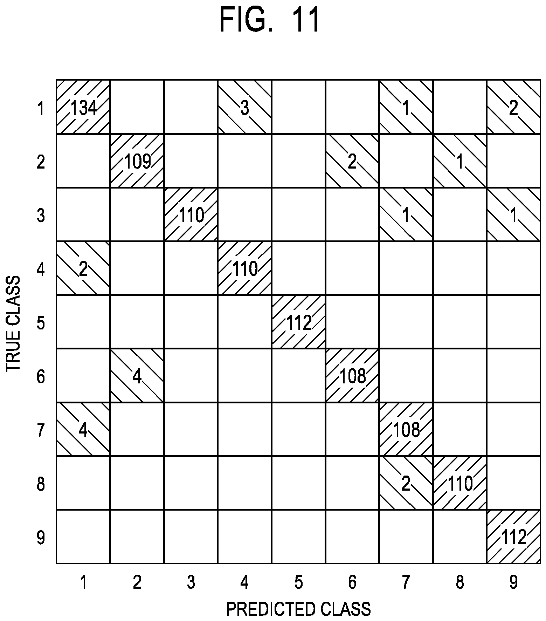

   Fig. 11 is a table illustrating a result of cross-validation.
[Fig. 12]

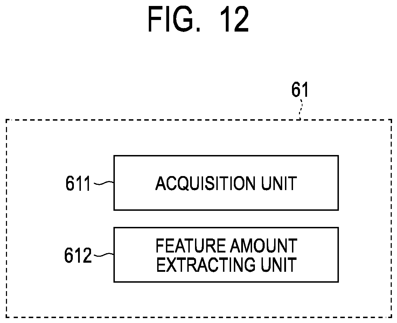

   Fig. 12 is a functional block diagram of an information processing device according to a second example embodiment.

### [Description of Embodiments]

Exemplary embodiments of the present invention are described below with reference to the drawings. Throughout the drawings, the same components or corresponding components are labeled with same references, and the description thereof may be omitted or simplified.

### [First Example Embodiment]

A personal identification system according to the present example embodiment is described. The personal identification system of the present example embodiment is a system for performing personal identification by measuring and analyzing features included in a walking pattern of a user (gait).

Fig. 1 is a schematic diagram illustrating a general configuration of a personal identification system according to the present example embodiment. The personal identification system includes a gait measurement device 1, an information communication terminal 2, a server 3, and load measurement devices 6a and 6b, which can be connected to each other by wireless communication. The load measurement device 6a may be referred to as a first load measurement device, and the load measurement device 6b may be referred to as a second load measurement device.

The gait measurement device 1 and the load measurement devices 6a and 6b are provided to be close to the sole of a shoe 5 worn by a user 4, for example. The gait measurement device 1 and the load measurement device 6a, and the gait measurement device 1 and the load measurement device 6b are communicatively connected by wiring or the like. The load measurement devices 6a and 6b are sensors for measuring load received from the sole of the user 4. The load measurement devices 6a and 6b convert load received from the user 4 into electrical signals and output the electrical signals to the gait measurement device 1 under the control of the gait measurement device 1. The load conversion method of the load measurement devices 6a and 6b may be a spring type, a piezoelectric element type, a magnetostrictive type, an electrostatic capacitance type, a gyro type, a strain gauge type, or the like, but is not particularly limited. The load measurement devices 6a and 6b are sometimes referred to as load cells. The gait measurement device 1 is an electronic apparatus having a control function of the load measurement devices 6a and 6b, an information processing function of analyzing measured load information, a communication function with the information communication terminal 2, or the like.

Note that, the gait measurement device 1 and load measurement devices 6a and 6b may be provided in the insole of the shoe 5, may be provided in the outsole of the shoe 5, or may be embedded in the shoe 5. The gait measurement device 1 and the load measurement devices 6a and 6b may be detachably attached to the shoe 5 or may be non-detachably fixed to the shoe 5. The gait measurement device 1 and the load measurement devices 6a and 6b may be provided at a portion other than the shoe 5 as long as the gait measurement device 1 can measure the load of the foot. For example, the gait measurement device 1 may be provided in a sock which the user 4 is wearing, provided in a decoration, directly attached to the foot of the user 4, or embedded in the foot of the user 4. Although Fig. 1 illustrates an example in which one gait measurement device 1 and two load measurement devices 6a and 6b are provided on one foot of the user 4, one gait measurement device 1 and two load measurement devices 6a and 6b may be provided on each of both feet of the user 4. In this case, the load information of both feet can be acquired in parallel, and more information can be acquired.

In this specification, the "foot" means a body part below an ankle of the user 4. In addition, in this specification, the "user" means a person who is an object of personal identification using the gait measurement device 1 and load measurement devices 6a and 6b. Whether or not the user corresponds to the "user" is unrelated to whether or not the user is a user of a device other than the gait measurement device 1 and load measurement devices 6a and 6b constituting the personal identification system, whether or not the user receives a service provided by the personal identification system, or the like.

The information communication terminal 2 is a terminal device carried by the user 4, such as a cellular phone, a smartphone, or a smart watch. Application software for analyzing information acquired from the gait measurement device 1 is installed in advance in the information communication terminal 2, and processing based on the application software is performed. The information communication terminal 2 acquires data acquired by the gait measurement device 1 and performs information processing using the data. The result of the information processing may be notified to the user 4 or may be transmitted to the server 3. The information communication terminal 2 may have a function of providing software such as a control program of the gait measurement device 1 or a data analysis program to the gait measurement device 1.

The server 3 provides and updates analysis application software to the information communication terminal 2. The server 3 may store data acquired from the information communication terminal 2 and perform information processing using the data.

Note that, the general configuration is an example, and for example, the gait measurement device 1 may be directly connected to the server 3. Further, the gait measurement device 1 and the information communication terminal 2 may be configured as an integrated device, and another device such as an edge server or a relay device may be further included in the personal identification system.

Fig. 2 is a schematic diagram illustrating an arrangement of load measurement devices 6a and 6b according to the present example embodiment. Fig. 2 is a perspective view of the shoe 5 viewed from the bottom side. The load measurement device 6a is provided at a position corresponding to the heel of the user 4, and the load measurement device 6b is provided between the toe and the load measurement device 6a. More specifically, the load measurement device 6a is provided between the position corresponding to the Lisfranc joint 7 of the foot (the joint between the metatarsal bone and the tarsal bone of the foot) and the heel, and the load measurement device 6b is provided between the position corresponding to the Lisfranc joint 7 of the foot and the toe. A dashed dotted line with reference numeral "7" in the figure indicates the position of the Lisfranc joint 7 when the user 4 wears the shoe 5.

Fig. 3 is a block diagram illustrating a hardware configuration example of the gait measurement device 1. The gait measurement device 1 is, for example, a microcomputer or a microcontroller. The gait measurement device 1 includes a central processing unit (CPU) 101, a random access memory (RAM) 102, a read only memory (ROM) 103, a flash memory 104, a communication interface (I/F) 105, a sensor control device 106, and a battery 107. Each unit in the gait measurement device 1 is connected each other via a bus, wiring, a driving device, or the like.

The CPU 101 is a processor that performs predetermined calculation in accordance with a program stored in the ROM 103, the flash memory 104, or the like, and also has a function of controlling each unit of the gait measurement device 1. The RAM 102 is composed of a volatile storage medium and provides a temporary memory area required for the operation of the CPU 101. The ROM 103 is composed of a non-volatile storage medium and stores necessary information such as a program used for the operation of the gait measurement device 1. The flash memory 104 is a storage device composed of a non-volatile storage medium and temporarily storing data, storing an operation program of the gait measurement device 1, or the like.

The communication I/F 105 is a communication interface based on standards such as Bluetooth (registered trademark) and Wi-Fi (registered trademark), and is a module for performing communication with the information communication terminal 2.

The sensor control device 106 is a control device that controls the load measurement devices 6a and 6b to measure load and acquires an electric signal indicating the load from the load measurement devices 6a and 6b. The acquired electrical signal is stored in the flash memory 104 as digital data. Thus, the gait measurement device 1 can acquire the load measured by the load measurement devices 6a and 6b as time series data. The load measured by the load measurement device 6a may be referred to as first load information, and the load measured by the load measurement device 6b may be referred to as second load information. The time series data of the load measured by the load measurement device 6a may be referred to as first time series data, and the time series data of the load measured by the load measurement device 6b may be referred to as second time series data. Note that analog-to-digital (AD) conversion for converting analog signals measured by the load measurement devices 6a and 6b into digital data may be performed in the load measurement devices 6a and 6b, or may be performed by the sensor control device 106.

The battery 107 is, for example, a secondary battery, and supplies power necessary for the operations of the gait measurement device 1. When power is required to be supplied to the load measurement devices 6a and 6b, the battery 107 may also supply power to the load measurement devices 6a and 6b. Since the battery 107 is built in the gait measurement device 1, the gait measurement device 1 can operate wirelessly without connecting to an external power source by wire.

Note that the hardware configuration illustrated in Fig. 3 is an example, and other devices may be added or some devices may not be provided. Further, some devices may be replaced by other devices having similar functions. For example, the gait measurement device 1 may further include an input device such as a button so that an operation by the user 4 can be accepted, and may further include an output device such as a display, a display lamp, and a speaker for providing information to the user 4. Thus, the hardware configuration illustrated in Fig. 3 can be changed appropriately.

Fig. 4 is a block diagram illustrating a hardware configuration example of the information communication terminal 2. The information communication terminal 2 includes a CPU 201, a RAM 202, a ROM 203, and a flash memory 204. The information communication terminal 2 also includes a communication I/F 205, an input device 206, and an output device 207. Each unit of the information communication terminal 2 is connected to each other via a bus, wiring, a driving device, or the like.

In Fig. 4, each unit constituting the information communication terminal 2 is illustrated as an integrated device, but some of these functions may be provided by an external device. For example, the input device 206 and the output device 207 may be external devices different from those constituting the functions of the computer including the CPU 201 or the like.

The CPU 201 is a processor that performs predetermined calculation in accordance with a program stored in the ROM 203, the flash memory 204, or the like, and also has a function of controlling each unit of the information communication terminal 2. The RAM 202 is composed of a volatile storage medium and provides a temporary memory area required for the operation of the CPU 201. The ROM 203 is composed of a non-volatile storage medium and stores necessary information such as a program used for the operation of the information communication terminal 2. The flash memory 204 is a storage device composed of a non-volatile storage medium for storing data transmitted and received to and from the gait measurement device 1 and for storing a program for operating the information communication terminal 2.

The communication I/F 205 is a communication interface based on standards such as Bluetooth (registered trademark), Wi-Fi (registered trademark), or 4G and is a module for performing communication with other devices.

The input device 206 is a user interface used by the user 4 to operate the information communication terminal 2. Examples of the input device 206 include a mouse, a trackball, a touch panel, a pen tablet, a button, or the like.

The output device 207 is, for example, a display device. The display device is a liquid crystal display, an organic light emitting diode (OLED) display, or the like, and is used for displaying information, displaying a graphical user interface (GUI) for operation input, or the like. The input device 206 and the output device 207 may be integrally formed as a touch panel.

Note that the hardware configuration illustrated in Fig. 4 is an example, and other devices may be added or some devices may not be provided. Further, some devices may be replaced by other devices having similar functions. Further, some functions of the present example embodiment may be provided by other devices via a network, or some functions of the present example embodiment may be realized by being distributed among a plurality of devices. For example, the flash memory 204 may be replaced by a hard disk drive (HDD) or a cloud storage. Thus, the hardware configuration illustrated in Fig. 4 can be changed appropriately.

The server 3 is a computer having substantially the same hardware configuration as that illustrated in Fig. 4. Since the hardware configuration of the server 3 is substantially the same as that of the information communication terminal 2 except that the server 3 may not be portable, a detailed description thereof is omitted.

Fig. 5 is a functional block diagram of the gait measurement device 1 according to the present example embodiment. The gait measurement device 1 includes an acquisition unit 120, a feature amount extracting unit 130, an identification unit 140, a storage unit 150, and a communication unit 160. The feature amount extracting unit 130 includes a time series data processing unit 131, a walking cycle identification unit 132, and a feature amount calculation unit 133.

The CPU 101 loads a program stored in the ROM 103, the flash memory 104, or the like into the RAM 102 and executes the program. Thus, the CPU 101 realizes the functions of the feature amount extracting unit 130 and the identification unit 140. Further, the CPU 101 realizes the function of the acquisition unit 120 by controlling the load measurement devices 6a and 6b based on the program. The CPU 101 realizes the function of the storage unit 150 by controlling the flash memory 104 based on the program. Further, the CPU 101 realizes the function of the communication unit 160 by controlling the communication I/F 105 based on the program. Specific processing performed by each of these units is described later.

In the present example embodiment, each function of the functional blocks illustrated in Fig. 5 is provided in the gait measurement device 1, but some functions of the functional blocks illustrated in Fig. 5 may be provided in the information communication terminal 2 or the server 3. That is, the above-described functions may be realized by any of the gait measurement device 1, the information communication terminal 2, and the server 3, or may be realized by cooperation of the gait measurement device 1, the information communication terminal 2, and the server 3. In the present example embodiment, since the gait measurement device 1 has a function of performing personal identification processing, the gait measurement device 1 may be referred to as a personal identification device more generally.

Fig. 6 is a flowchart illustrating an example of personal identification processing performed by the gait measurement device 1 according to the present example embodiment. The process of Fig. 6 is performed when the gait measurement device 1 detects walking, for example, when the user 4 is walking. Alternatively, the process of Fig. 6 may be always performed unrelated to whether or not the user 4 is walking, or may be performed at predetermined time intervals.

In step S101, the acquisition unit 120 controls the load measurement devices 6a and 6b to acquire time series data of load from the load measurement devices 6a and 6b. That is, the acquisition unit 120 acquires the first time series data from the load measurement device 6a and acquires the second time series data from the load measurement device 6b. Thus, the acquisition unit 120 can acquire time changes in the load caused by walking of the user 4. The acquired time series data of the load is converted into digital data and then stored in the storage unit 150. In addition, the time series data of the load is referred to as walking data because it indicates the feature of walking. The walking data can be used not only for the personal identification processing of the present example embodiment but also for the gait analysis of the user 4.

Here, in order to sufficiently acquire features included in walking, it is desirable that time series data of the load include data in a period corresponding to at least one walking cycle. One walking cycle is described with reference to Fig. 7. Fig. 7 is a conceptual diagram illustrating a walking cycle. Fig. 7 schematically illustrates motion of the right foot and the left foot of the user 4 for one walking cycle. The normalized time in the figure indicates the time normalized so that the length of one walking cycle is 100. That is, the normalized time 0 in the figure is the moment at which the right foot lands, the normalized time 50 in the figure is the moment at which the left foot lands, and the normalized time 100 in the figure is the moment at which the right foot lands again. A period from the normalized time 0 to 100 is one walking cycle.

Further, a period in which the foot lands is referred to as a stance period, and a period in which the foot leaves the ground is referred to as a swing period. More specifically, for example, the stance period of the right foot is a period from the moment at which the heel of the right foot lands (at the time of landing) to the moment at which the toe of the right foot leaves the ground (at the time of leaving), and generally occupies a period of about 60% of one walking cycle. The swing period of the right foot is a period from the moment when the toe of the right foot leaves the ground to the moment when the heel of the right foot lands, and generally occupies a period of about 40% of one walking cycle. As illustrated in Fig. 7, during walking, the stance period and the swing period are alternately repeated. Further, the phase of the stance period and the phase of the swing period are opposite between the right foot and the left foot.

In step S102, the time series data processing unit 131 generates third time series data by adding the first time series data acquired from the load measurement device 6a and the second time series data acquired from the load measurement device 6b. In this process, digital data of load at the same time in the first time series data and the second time series data is added together. Thus, the third time series data including the feature based on the load output from both the load measurement devices 6a and 6b can be acquired.

The order and contents of step S101 and step S102 can be changed appropriately. Step S101 and step S102 may be performed simultaneously or as a series of processes. For example, these processes may be modified to a process in which the time series data is added together to generate and store third time series data when the time series data is acquired from the load measurement devices 6a and 6b. In this case, the first time series data and the second time series data are not necessary to be stored. These processes may be modified to a process of adding analog signals measured by the load measurement devices 6a and 6b in a circuit before AD conversion. In this case, the addition is completed before the acquisition unit 120 acquires the data. In this case, the number of AD conversion processes is reduced.

In step S103, the walking cycle identification unit 132 identifies one walking cycle from the third time series data. Since substantially the same motion is repeated for each step during walking, one walking cycle can be identified by detecting periodicity of the third time series data. For example, one walking cycle can be identified based on the appearance time of a peak or dip of the third time series data, a frequency of a peak included in a frequency spectrum acquired by Fourier-transforming the third time series data, or the like.

In step S104, the feature amount calculation unit 133 extracts a feature amount used for identifying the user 4 from the third time series data of at least one walking cycle. The extracted feature amount is stored in the storage unit 150. The extraction of the feature amount is described with a specific example.

Fig. 8 is a graph illustrating an example of time series data of load. The horizontal axis of Fig. 8 represents time, and the vertical axis of Fig. 8 represents load in arbitrary unit.

Three graphs of the different line types illustrate the time changes in load measured from three different subjects (subject 1, subject 2, subject 3). As understood from Fig. 8, in any of the three subjects, steep peaks (P1, P2) appears at the beginning and end of the stance period, and a steep dip (V) appears between them. In addition, the differences between the three graphs become large in the stance period, and become significantly large in the vicinity of the peak and dip. Therefore, the subject can be identified by extracting the feature amount from the vicinity of the peaks at the beginning and end of the stance period and the vicinity of the dip between them in time series data of the load.

The reason why the features appear in the peak and dip of the stance period is described. During human walking, in the stance period, the load concentration points are shifted in the order of the tarsal bone, the metatarsal bone, and the phalanx bone. In other words, at the beginning of the stance period (that is, immediately after the foot lands on the ground), the load is concentrated on the heel of the foot. At the end of the stance period (that is, just before the foot leaves the ground), load is concentrated on the toe of the foot. In the beginning and end of the stance period and in the period between them, individual differences are likely to occur in the load and the intensity of the signal is large, so that the method is suitable for extracting the feature amount. In particular, as illustrated in Fig. 8, since the features strongly appear in the peaks and dips in the stance period, they are particularly suitable for extracting feature amount. These peaks occur twice within one walk cycle and dips occur once within one walk cycle. In order to make use of the peak and dip of the stance period for feature extraction, in the present example embodiment, it is desirable to acquire time series data of at least one walking cycle and extract a feature amount.

The appearance time, intensity, or the like of the peak or dip may be used as a feature amount. Further, statistical amount acquired by performing statistical calculations such as a sum, an average value, a difference, a ratio, and a product on the appearance time, intensity, and the like of a peak or dip may be extracted as feature amounts. The feature amount extracted in this process may include a plurality of elements, in other words, the feature amount extracted in this process may be a feature amount vector.

In step S105, the identification unit 140 identifies the user 4 based on the extracted feature amount. This identification process may be a process of determining whether or not the user 4 is the same person as a person registered in the gait measurement device 1 in advance (registrant). A specific example of this process is to compare the acquired feature amount with the feature amount of the registrant to calculate a score indicating a certainty factor, and to determine whether or not the user 4 is the same person as the registrant based on whether or not the score exceeds a predetermined threshold value.

In the process of identifying the user 4 from the feature amount performed by the identification unit 140, a trained model generated in advance by machine learning and stored in the storage unit 150 is used. Examples of algorithms used for machine learning include decision trees, random forests, support vector machines, neural networks, deep learning, logistic regression, k-nearest neighbor algorithm (K-NN), ensemble learning for classification method, discriminant analysis, or the like. Further, generation of a trained model by machine learning (training process) is performed in the gait measurement device 1, the information communication terminal 2, or the server 3 using sample data prepared in advance.

The training process for generating a trained model used for personal identification in step S105 is described in more detail. This process is performed in advance in the gait measurement device 1, the information communication terminal 2, or the server 3 prior to the process of Fig. 6. In the description of the present example embodiment, it is assumed that the training process is performed in the server 3.

Fig. 9 is a flowchart illustrating an example of training process performed by the server 3 according to the present example embodiment. The process of Fig. 9 is performed prior to the personal identification process at the time of shipment from a factory, calibration before the user 4 uses the gait measurement device 1, or the like.

In step S201, the server 3 acquires sample data for training. This sample data may be, for example, one in which a personal identification label (user identifier (ID), name, nickname, or the like) for identifying a person is associated with a feature amount vector acquired by the processing from step S101 to step S104. The personal identification label is attached in advance by the user 4, the administrator of the personal identification system, or the like. More specifically, by causing the user 4 to actually walk a predetermined distance after being input his/her personal identification label to the gait measurement device 1 and causing the gait measurement device 1 to acquire data, sample data in which feature amount vectors and personal identification labels are associated with each other can be created.

In step S202, the server 3 performs machine learning on the sample data as labeled training data. As a result, a trained model is generated in which a person is appropriately identified with respect to the input of the feature amount vector and the identification information is output.

In step S203, the server 3 stores the trained model in the flash memory 204. Thereafter, the server 3 provides the trained model to the gait measurement device 1. Specifically, the server 3 transmits the trained model to the information communication terminal 2. The information communication terminal 2 causes the gait measurement device 1 to install the received trained model as software for processing in the identification unit 140.

Fig. 10 is a table schematically illustrating the correspondence relation between a feature vector and a personal identification label acquired by this training process. As illustrated in Fig. 10, a personal identification label is determined corresponding to a feature amount vector including the intensity of the peak P1 (P1), the intensity of the peak P2 (P2), the intensity ratio of the peak P2 and the dip V (P2/V), and the like. In other words, the trained model acquired by the training process has a function of outputting a personal identification label as a response variable when a feature amount vector is input as an explanatory variable.

Hereinafter, the results of actually performing personal identification using the personal identification system of the first example embodiment are described as Example 1.

### [Example 1]

In the present example, the load information during walking was measured for nine subjects, and walking data was acquired. A large number of feature amount vectors were extracted from these walking data to create data groups for training and validation. Specifically, some randomly selected data in the data group was used as validation data, and the remaining data was used as training data. That is, a trained model was generated using training data of a part of the data group, and the recognition rate of the trained model was validated using the remaining data. The machine learning algorithm used in the present example embodiment was a random forest.

Fig. 11 is a table illustrating results of cross-validation using these data groups. The "predicted class" in the table is a class of a subject determined by the personal identification system of the first example embodiment, and the "true class" is an actual class indicating a subject. The class numbers "1" to "9" in the table indicate subject numbers. For example, in the prediction performed by the personal identification system for 112 data groups whose true class is "4", the personal identification system correctly predicted the class "4" for 110 of the 112 data groups. In contrast, an incorrect class "1" was predicted for two of 112. As illustrated in Fig. 11, in the personal identification system of the first example embodiment, the subject could be correctly determined at a high correct answer rate of 97.7% or more.

The gait measurement device 1 of the present example embodiment acquires feature amounts for personal identification using two kinds of load information acquired from the two load measurement devices 6a and 6b. Here, the two load measurement devices 6a and 6b are arranged at different positions along the front/back direction of the foot so as to be able to detect the transition of the load concentration point during walking. As a result, personal identification with sufficient accuracy can be performed by the feature amounts acquired from the two load measurement devices 6a and 6b. Therefore, the amount of data can be reduced as compared with a method requiring a large amount of data such as a method of acquiring an in-plane distribution of load.

As described above, according to the present example embodiment, it is possible to provide an information processing device capable of reducing the amount of data in extracting feature amounts used for personal identification. In addition, it is possible to provide a personal identification device and a personal identification system capable of performing personal identification with high accuracy by using the feature amount extracted by the information processing device.

As illustrated in Fig. 2, it is desirable that the two load measurement devices 6a and 6b be provided between the heel and the Lisfranc joint and between the toe and the Lisfranc joint, respectively. By arranging in this way, it is possible to more reliably extract the feature of the load which is transferred in the order of the tarsal bone, the metatarsal bone, and the phalanx bone.

The personal identification system of the present example embodiment can identify the user 4 wearing the shoe 5 provided with the personal identification device. Hereinafter, an example of application of the personal identification system of the present example embodiment is described.

In recent years, biometric authentication using biometric information has been widely used in personal authentication. Examples of the biological information include a face, a fingerprint, a voiceprint, and an iris. However, depending on where biometric authentication is performed, biometric authentication using them may not be effective.

For example, in facilities requiring wearing of protective clothes, masks, or the like, such as food plants, it is difficult to use authentication techniques using them because parts for acquiring biological information such as a face and a fingerprint are not exposed to the outside. Further, in facilities in which safety and health are severely required, such as in chemical plants, installation of a device for biometric authentication may be limited. In a facility where security is severely required, such as a power plant, authentication may be desired without informing the subject that biometric authentication is being performed. In addition, although it is required to continue authentication of a subject at all times, such as a care facility, there is a facility in which a blind spot exists in a biometric authentication device such as a camera. In addition, when it is necessary to always specify a subject, it is difficult to use an authentication technique which requires the subject to perform an action on an input device such as a camera, a sensor, or a microphone.

Thus, depending on the location where biometric authentication is performed and the constraints required for biometric authentication, biometric authentication using a face, a fingerprint, a voiceprint, iris, or the like may not be effective.

In contrast, the biometric information included in the gait can be acquired even if a part such as a face or a fingerprint of the subject is not exposed to the outside. Further, the authentication device using the gait can be installed in the shoe, so that it is not necessary to install the biometric authentication device outside, and it is also possible to perform the biometric authentication without informing the subject that the biometric authentication is being performed. The gait can be measured at all times, and it is not necessary to request actions other than walking in the measurement. Therefore, the personal identification system of the present example embodiment can perform personal identification with high accuracy even in a place where biometric authentication such as a face, a fingerprint, a voiceprint, or an iris is not effective.

The device or system described in the above example embodiment can also be configured as in the following second example embodiment.

### [Second Example Embodiment]

Fig. 12 is a functional block diagram of the information processing device 61 according to the second example embodiment. The information processing device 61 includes an acquisition unit 611 and a feature amount extracting unit 612. The acquisition unit 611 acquires first load information measured by a first load measurement device provided on a sole of a user and second load information measured by a second load measurement device provided between a toe of the sole and the first load measurement device. The feature amount extracting unit 612 extracts a feature amount used for identifying the user based on the first load information and the second load information.

According to the present example embodiment, an information processing device 61 that can reduce the amount of data in extracting feature amounts used for personal identification is provided.

### [Modified Example Embodiments]

The present invention is not limited to the example embodiments described above, and may be suitably modified within the scope of the present invention. For example, an example in which a part of the configuration of one example embodiment is added to another example embodiment or an example in which a part of the configuration of one example embodiment is replaced with another example embodiment is also an example embodiment of the present invention.

In the above-described embodiments, the load measurement devices 6a and 6b are provided in the gait measurement device 1 as an example, and sufficient authentication accuracy can be acquired only by these devices, so that the amount of data can be reduced. However, in a case where priority is given to authentication accuracy rather than reduction of the amount of data, sensors other than these may be further used. For example, an angular velocity sensor for measuring angular velocity in three axial directions, an acceleration sensor for measuring acceleration in three directions, and a magnetic sensor that detects geomagnetism by detecting magnetism in three directions to identify an azimuth may be further used. In addition, more than two load measurement devices may be provided. Even in these cases, the same processing as the above-described example embodiments can be applied, and the accuracy can be further improved.

Although the personal identification process is performed inside the gait measurement device 1 in the above-described example embodiments, this function may be provided in the information communication terminal 2. In this case, the information communication terminal 2 functions as a personal identification device.

In the above-described example embodiments, the feature amount may be extracted from the time series data or the time series data may be performed processing such as Fourier transform to acquire a frequency spectrum and extract the feature amount from the frequency spectrum.

Although the feature amount is extracted from the third time series data acquired by adding the first time series data and the second time series data in the above-described example embodiments, the feature amount may be extracted from the first time series data and the second time series data. In this case, the addition process can be omitted. Alternatively, the feature amount extracted from the first time series data or the second time series data and the feature amount extracted from the third time series data may be combined and used for personal identification.

A processing method in which a program for operating the configuration of the above-described example embodiments is recorded in a storage medium so as to implement the functions of the above-described example embodiments, the program recorded in the storage medium is read as code, and the program is executed in a computer is also included in the scope of each example embodiment. That is, a computer-readable storage medium is also included in the scope of the example embodiments. Further, not only the storage medium in which the above program is recorded, but also the program itself is included in each example embodiment. In addition, one or more components included in the above-described example embodiments may be a circuit such as an application specific integrated circuit (ASIC), a field programmable gate array (FPGA) configured to implement the functions of each component.

As the storage medium, for example, a floppy (registered trademark) disk, a hard disk, an optical disk, a magneto-optical disk, a compact disk (CD)-ROM, a magnetic tape, a nonvolatile memory card, or a ROM can be used. Further, the scope of each example embodiment is not limited to the case where the processing is executed by the program alone recorded in the storage medium, and a case where the processing is executed by operating on an operating system (OS) in cooperation with the functions of other software and extension board is also included in the scope of each example embodiment.

The service realized by the functions of the above-described example embodiments may be provided to the user in the form of a software as a service (SaaS).

It should be noted that the above-described example embodiments are merely examples of embodying the present invention, and the technical scope of the present invention should not be limitedly interpreted by these. That is, the present invention can be implemented in various forms without departing from the technical idea or the main features thereof.

The whole or part of the example embodiments disclosed above can be described as, but not limited to, the following supplementary notes.

### (Supplementary note 1)

An information processing device comprising:
an acquisition unit configured to acquire first load information measured by a first load measurement device provided on a sole of a user and second load information measured by a second load measurement device provided between a toe of the sole and the first load measurement device; and
a feature amount extracting unit configured to extract a feature amount used for identifying the user based on the first load information and the second load information.

### (Supplementary note 2)

The information processing device according to supplementary note 1,
wherein the first load information includes first time series data in a period corresponding to at least one walking cycle of the user; and
wherein the second load information includes second time series data in a period corresponding to the at least one walking cycle.

### (Supplementary note 3)

The information processing device according to supplementary note 2, wherein the feature amount extracting unit extracts the feature amount based on a feature within a stance period in the first time series data and the second time series data.

### (Supplementary note 4)

The information processing device according to supplementary note 2 or 3, wherein the feature amount extracting unit extracts the feature amount based on third time series data acquired by adding the first time series data and the second time series data.

### (Supplementary note 5)

The information processing device according to supplementary note 4, wherein the feature amount extracting unit extracts the feature amount based on at least one of a peak and a dip included in the third time series data.

### (Supplementary note 6)

The information processing device according to supplementary note 4 or 5, wherein the feature amount extracting unit extracts the feature amount based on two peaks and one dip included in the third time series data.

### (Supplementary note 7)

The information processing device according to any one of supplementary notes 1 to 6,
wherein the first load measurement device is provided between a heel and a Lisfranc joint of a foot of the user, and
wherein the second load measurement device is provided to between a toe and the Lisfranc joint.

### (Supplementary note 8)

The information processing device according to any one of supplementary notes 1 to 7, wherein the feature amount extracting unit extracts the feature amount based on only the first load information and the second load information.

### (Supplementary note 9)

A personal identification device configured to perform an identification of the user based on a feature amount extracted by the information processing device according to any one of supplementary notes 1 to 8.

### (Supplementary note 10)

A personal identification system comprising:
the information processing device according to any one of supplementary notes 1 to 8;
an identification unit configured to identify the user based on the feature amount;
the first load measurement device; and
the second load measurement device.

### (Supplementary note 11)

An information processing method comprising:
acquiring first load information measured by a first load measurement device provided on a sole of a user and second load information measured by a second load measurement device provided between a toe of the sole and the first load measurement device; and
extracting a feature amount used for identifying the user based on the first load information and the second load information.

### (Supplementary note 12)

A storage medium storing a program that causes a computer to perform:
acquiring first load information measured by a first load measurement device provided on a sole of a user and second load information measured by a second load measurement device provided between a toe of the sole than the first load measurement device; and
extracting a feature amount used for identifying the user based on the first load information and the second load information.

### [Reference Signs List]

1 gait measurement device
2 information communication terminal
3 server
4 user
5 shoe
6a, 6b load measurement device
7 Lisfranc joint
61 information processing device
101, 201 CPU
102, 202 RAM
103, 203 ROM
104, 204 flash memory
105, 205 communication I/F
106 sensor control device
107 battery
120, 611 acquisition unit
130, 612 feature amount extracting unit
131 time series data processing unit
132 walking cycle identification unit
133 feature amount calculation unit
140 identification unit
150 storage unit
160 communication unit
206 input device
207 output device

## Claims

1. An information processing device comprising:
an acquisition unit configured to acquire first load information measured by a first load measurement device provided on a sole of a user and second load information measured by a second load measurement device provided between a toe of the sole and the first load measurement device; and
a feature amount extracting unit configured to extract a feature amount used for identifying the user based on the first load information and the second load information.

2. The information processing device according to claim 1,
wherein the first load information includes first time series data in a period corresponding to at least one walking cycle of the user; and
wherein the second load information includes second time series data in a period corresponding to the at least one walking cycle.

3. The information processing device according to claim 2, wherein the feature amount extracting unit extracts the feature amount based on a feature within a stance period in the first time series data and the second time series data.

4. The information processing device according to claim 2 or 3, wherein the feature amount extracting unit extracts the feature amount based on third time series data acquired by adding the first time series data and the second time series data.

5. The information processing device according to claim 4, wherein the feature amount extracting unit extracts the feature amount based on at least one of a peak and a dip included in the third time series data.

6. The information processing device according to claim 4 or 5, wherein the feature amount extracting unit extracts the feature amount based on two peaks and one dip included in the third time series data.

7. The information processing device according to any one of claims 1 to 6,
wherein the first load measurement device is provided between a heel and a Lisfranc joint of a foot of the user, and
wherein the second load measurement device is provided to between a toe and the Lisfranc joint.

8. The information processing device according to any one of claims 1 to 7, wherein the feature amount extracting unit extracts the feature amount based on only the first load information and the second load information.

9. A personal identification device configured to perform an identification of the user based on a feature amount extracted by the information processing device according to any one of claims 1 to 8.

10. A personal identification system comprising:
the information processing device according to any one of claims 1 to 8;
an identification unit configured to identify the user based on the feature amount;
the first load measurement device; and
the second load measurement device.

11. An information processing method comprising:
acquiring first load information measured by a first load measurement device provided on a sole of a user and second load information measured by a second load measurement device provided between a toe of the sole and the first load measurement device; and
extracting a feature amount used for identifying the user based on the first load information and the second load information.

12. A storage medium storing a program that causes a computer to perform:
acquiring first load information measured by a first load measurement device provided on a sole of a user and second load information measured by a second load measurement device provided between a toe of the sole than the first load measurement device; and
extracting a feature amount used for identifying the user based on the first load information and the second load information.
